# EUROPÄISCHE PATENTSCHRIFT

(11) **EP 1 440 148 B1**
(45) Veröffentlichungstag und Bekanntmachung des Hinweises auf die Patenterteilung: **14.07.2010**
(21) Anmeldenummer: 02781248.6
(22) Anmeldetag: 14.10.2002
(51) Int. Cl.: C12N 5/07

(54) **KERATINOZYTEN VERWENDBAR ALS BIOLOGISCH AKTIVE SUBSTANZ BEI DER BEHANDLUNG VON WUNDEN**
KERATINOCYTES WHICH MAY BE USED AS BIOLOGICALLY ACTIVE SUBSTANCES FOR THE TREATMENT OF WOUNDS
KERATINOCYTES S'UTILISANT COMME SUBSTANCE BIOLOGIQUEMENT ACTIVE DANS LE TRAITEMENT DE PLAIES

(30) Priorität: 17.10.2001 DE 10151296
(43) Veröffentlichungstag der Anmeldung: 28.07.2004
(73) Patentinhaber: Boehringer Ingelheim Pharma GmbH & Co. KG, 55216 Ingelheim am Rhein (DE)
(72) Erfinder: EBERHARDT, Petra, 88400 Biberach (DE); NOE, Wolfgang, San Diego, CA 92130 (US); REIF, Katharina, 86391 Stadtbergen (DE)
(86) Internationale Anmeldenummer: PCT/EP2002/011459
(87) Internationale Veröffentlichungsnummer: WO 2003/033686

(56) Entgegenhaltungen:
- EP-A- 0 462 426
- WO-A-93/10217
- WO-A-96/24018
- WO-A-99/47644
- DE-A- 19 917 532
- PELLEGRINI G ET AL: "Location and Clonal Analysis of Stem Cells and their Differentiated Progeny in the Human Ocular Surface" THE JOURNAL OF CELL BIOLOGY, ROCKEFELLER UNIVERSITY PRESS, US, Bd. 145, Nr. 4, 17. Mai 1999 (1999-05-17), Seiten 769-782, XP002192318 ISSN: 0021-9525
- PELLEGRINI GRAZIELLA ET AL: "p63 identifies keratinocyte stem cells" PROCEEDINGS OF THE NATIONAL ACADEMY OF SCIENCES OF USA, NATIONAL ACADEMY OF SCIENCE. WASHINGTON, US, Bd. 98, Nr. 6, 13. März 2001 (2001-03-13), Seiten 3156-3161, XP002192317 ISSN: 0027-8424
- PELLEGRINI G ET AL: "CULTIVATION OF HUMAN KERATINOCYTE STEM CELLS: CURRENT AND FUTURE CLINICAL APPLICATIONS" MEDICAL AND BIOLOGICAL ENGINEERING AND COMPUTING, PETER PEREGRINUS LTD. STEVENAGE, GB, Bd. 36, Nr. 6, 1. November 1998 (1998-11-01), Seiten 778-790, XP000784853 ISSN: 0140-0118
- HARLE-BACHOR C ET AL: "TELOMERASE ACTIVITY IN THE REGENERATIVE BASAL LAYER OF THE EPIDERMIS IN HUMAN SKIN AND IN IMMORTAL AND CARCINOMA-DERIVED SKIN KERATINOCYTES" PROCEEDINGS OF THE NATIONAL ACADEMY OF SCIENCES OF USA, NATIONAL ACADEMY OF SCIENCE. WASHINGTON, US, Bd. 93, Juni 1996 (1996-06), Seiten 6476-6479,6481, XP002923264 ISSN: 0027-8424
- WEAVER J D ET AL: "PARTIAL TRISOMIES IN TWO SPONTANEOUSLY ARISING LONG-LIVED HUMAN KERATINOCYTE LINES" IN VITRO CELLULAR & DEVELOPMENTAL BIOLOGY, Bd. 27A, Nr. 8, 1991, Seiten 670-675, XP009017082 ISSN: 0883-8364
- BOUKAMP P ET AL: "NORMAL KERATINIZATION IN A SPONTANEOUSLY IMMORTALIZED ANEUPLOID HUMAN KERATINOCYTE CELL LINE" JOURNAL OF CELL BIOLOGY, ROCKEFELLER UNIVERSITY PRESS, NEW YORK, US, US, Bd. 106, Nr. 3, März 1988 (1988-03), Seiten 761-771, XP000867527 ISSN: 0021-9525

## Beschreibung

### Feld der Erfindung

Die Erfindung betrifft neue *in vitro* kultivierbare Keratinozyten so wie deren vorteilhafte Verwendung zur Herstellung eines Erzeugnisses, welches zur Behandlung von akuten und chronischen Wunden verwendet werden kann. Die Erfindung erfolgte somit auf dem Gebiet der Medizin, speziell auf dem Gebiet der Wundheilung mittels *Tissue Engineering.*

### Stand der Technik

Allogene Keratinozyten werden seit längerem erfolgreich zur Behandlung von Wunden, insbesondere von Geschwüren und/oder Verbrennungen eingesetzt (Maier, 1993; Schönfeld et al., 1993). Wunden, die sich gegenüber herkömmlichen Therapien über einen lange Zeitraum therapieresistent zeigen, lassen sich unter Verwendung von allogenen Keratinozyten sehr oft erfolgreich therapieren (Phillips and Gilchrest, 1993; Beele et al., 1991; Leigh et al., 1991; Lindgren et al, 1998). Zu Beginn war das Augenmerk bei der Verwendung allogener Keratinozyten vornehmlich auf den Hautersatz, d.h. auf die Regeneration der transplantierten Epidermis gerichtet. Es zeigte sich jedoch bald, dass der Therapieerfolg primär auf der Stimulation der körpereigenen Reepithelisierung beruht und nicht auf einem "Anwachsen" des allogenen Zelltransplantats in der Wunde. Zahlreiche Untersuchungen ergaben letztlich, dass die transplantierten Keratinozyten nur eine bestimmte Zeit in der Wunde verbleiben und dann von dem Körper klinisch nicht sichtbar eliminiert werden (Kawai et al., 1993). Die Stimulation der körpereigenen Wundheilung erfolgt also primär über eine räumlich und zeitlich optimale Freisetzung von einer Vielzahl an unterschiedlichen Wachstumsfaktoren, Zytokinen, Extrazellulärer Matrix (ECM), kleineren Moleküle und Proteasen durch die allogenen Keratinozyten (Lang et al., 1996; Marcusson et al., 1992). Die Komplexität und Vielzahl der freigesetzten Faktoren begründet hierbei den therapeutischen Vorteil gegenüber herkömmlichen Einzeltherapien, z.B. mit isolierten Wachstumsfaktoren. Neben dem Haupteffekt der Reepithelisierung treten durch Behandlung mit Keratinozyten aber auch makroskopisch und mikroskopisch nachweisbare Veränderungen im Granulationsgewebe auf (Lang et al., 1996). Ein weiterer, für den Patienten wohltuender und oft beschriebener Effekt der Keratinozyten betrifft die ausgeprägte Analgesie nach Transplantation (Schönfeld et al., 1993).

Für die Wundheilung ist die Verwendung undifferenzierter, teilungsaktiver Keratinozyten vorteilhaft, denn teilungsaktive Keratinozyten scheinen über ein für die Wundheilung begünstigendes komplexes Sezernierungsprofil zu verfügen. Mit Ausnahme der Stammzellen verlieren undifferenzierte Keratinozyten *in vivo* ihr Proliferationspotential im Zuge der natürlicher Ausdifferenzierung bereits nach wenigen Zellteilungen, ein Prozess der bei der *in vitro* Kultivierung von Keratinozyten bisher immer beobachtet wurde (Barrandon and Green, 1987). Folglich lassen sich die bisher für die Wundheilung besonders geeigneten teilungsaktiven Keratinozyten nur endlich *in vitro* kultivieren, was ihre medizinische Verwendung aufwendig und kostenintensiv macht.

Die allogenen Keratinozyten werden entweder direkt in Form sog. "Keratinozyten-Sheets", bestehend aus einem mehrschichtigen, enzymatisch abgelösten Zellverbund oder zusammen mit biokompatiblen Trägern unter der Bezeichnung "biologisch aktive Wundheilverbände" therapeutisch eingesetzt. Letzteres hat den Vorteil, dass die Zellen nicht enzymatisch vorbehandelt werden müssen, indem sie vor ihrer Verwendung vom Untergrund der Kulturschale abgelöst werden. Darüber hinaus ermöglicht die Verwendung von biokompatibler Membranen als Träger zur Kultivierung der Keratinozyten (EP 0 462 462, US 5,658,331; US 5,693,332; EP 0518 920) eine zeitlich frühere Fertigstellung der biologisch aktiven Wundheilverbände, da die Zellen keinen geschlossen Zellverband mehr ausbilden müssen. Es lassen sich bereits subkonfluent bewachsene Träger zur Wundbehandlung einsetzten. Neben einer früheren Verfügbarkeit hat die Verwendung subkonfluenter Zellverbände zusätzlich den Vorteil, dass entsprechend kultivierte Keratinozyten weniger stark ausdifferenziert sind und somit die vorteilhaft für Behandlung von Wunden sind.

Keratinozyten lassen sich ohne nachteiligen Effekt für die Wundheilung direkt oder in Verbindung mit biokompatiblen Trägermaterialien bei Temperaturen zwischen -30 und -196°C, bevorzugt jedoch zwischen -70 und -90°C kryokonservieren (De Luca et al., 1992; Teepe et al., 1993). Dies hat ihre therapeutische Nutzbarkeit weiter verbessert, da sich biologisch aktive Wundheilverbände im gewissen Umfang auf Vorrat herstellen lassen.

### Aufgabe der Erfindung

Die dem Stand der Technik bekannten Keratinozyten sowie aus diesen hergestellte biologisch aktive Wundheilverbände verfügen derzeit über gewisse Nachteile, deren Überwindung Ziel der vorliegenden Erfindung war.

Ein offensichtlicher Nachteil der bisher bekannten Keratinozyten primären Ursprungs besteht darin, dass sich die besagten Zellen durch *in vitro* Zellkulturverfahren nur wenige Zellgenerationen verdoppeln lassen ohne dabei ihre hohe Teilungsaktivität und somit ihre bevorzugte Eignung zur Herstellung biologisch aktiver Wundheilverbände zu verlieren. Nach dem Stand der Technik ist es dem Fachmann lediglich möglich die Zahl der Zellen mittels *in vitro* Kultivierung um etwa das 10³ bis 10⁴-fache zu vermehren (Tanczos et al., 1999).

Ein weiterer, hierdurch bedingter Nachteil besteht darin, dass die begrenzte *in vitro* Kultivierbarkeit der besagten Keratinozyten eine häufige und aufwändige Neuisolierung erforderlich macht; was wiederum zur Folge hat, dass das gewonnene und vermehrte Zellmaterial nicht einheitlich ist und somit die hieraus hergestellten Wundheilverbände mit hoher Wahrscheinlichkeit Qualitätsunterschiede aufweisen oder zumindest aufweisen können.

Ein weiterer Nachteil besteht darin, dass mit der Neuisolierung der Keratinozyten aus verschiedenen Spendern ein erhöhte Infektionsrisiko für den Empfänger des Wundheilverbandes besteht. Zu denken ist hier beispielsweise an ein erhöhtes HIV- oder Hepatitis-Risiko.

### Beschreibung der Erfindung

Gegenstand der Erfindung sind Keratinozyten **dadurch gekennzeichnet, dass** sie nicht immortalisiert sind und sich durch *in vitro* Zellkulturverfahren mindestens 150 mal verdoppeln lassen und dass es sich dabei um Zellen aus der Kultur KC-BI-1 (DSM ACC 2514) handelt, oder um hiervon abgeleitete Keratinozyten, wobei die besagten Keratinozyten
a. nicht in Abwesenheit von Fötalem Kälber Serum und/oder
b. nicht in Abwesenheit von *Feeder*-Zellen und/oder
c. nicht in Abwesenheit von Epidermalem Wachstumsfaktor (EGF) verdoppelt werden können,
und wobei die Telomeraseaktivität der besagten Keratinozyten im Vergleich zu der Zellinie HaCaT um mindestens Faktor 2 niedriger ist,
und wobei abgeleitete Keratinozyten Zellen und Kulturen sind, die durch Subpassagieren und / oder Subklonieren der ursprünglichen Kultur KC-BI-1 (DSM ACC 2514) generiert werden und/ oder generiert werden können. Hieraus resultiert eine Zellvermehrung um ungefähr das 10⁴⁴-fache. Die entsprechenden Keratinozyten behalten hierbei ihre vorteilhaften Eigenschaften für die Behandlung von Wunden.

Bei den erfindungsgemäßen Keratinozyten handelt es sich um primär aus einem Spender isolierte und *in vitro* kultivierbare Keratinozyten, wobei die Isolierung und anfängliche Kultivierung von einem Fachmann beispielsweise entsprechend dem von Rheinwald und Green 1975 beschriebenen Verfahren ausgeführt werden kann.

Die Erfindung betrifft Keratinozyten, die aus dem epidermalen Anteil einer Vorhaut isoliert werden. Keratinozyten humanen Ursprungs, der Kultur KC-BI-1, wurden am 27.Juni 2001 bei der DSMZ-Deutsche Sammlung von Mikroorganismen und Zellkulturen GmbH, Braunschweig, Deutschland unter der Hinterlegungsnummer DSM ACC2514 für die Zwecke von Patentverfahren gem. Budapester Vertrag hinterlegt. Unter die Erfindung fallen auch solche Keratinozyten, die sich von der Kultur KC-BI-1 (DSM ACC2514) ableiten. Gegenstand der Erfindung sind somit auch alle Zellen und Kulturen, die durch Subpassagieren und/oder Subklonieren der ursprünglichen Kultur KC-BI-1 generiert werden und/oder generiert werden können.

Die erfindungsgemäße Kultivierung der Keratinozyten ist beispielhaft für die Keratinozyten KC-BI-ldargestellt (Beispiel 1). Vorteilhaft für die Kultivierung ist die Verwendung des in Beispiel 1 angegebenen komplexen Mediums sowie die Verwendung von *Feeder*-Zellen (engl. *feeder cells,* deutsch: Ammenzellen), bevorzugt die Verwendung von letal bestrahlten murinen 3T3 Fibroblasten. Der Anteil an fötalem Kälberserum sollte zwischen 2 und 10 % betragen. Die Herstellung der *Feeder*-Zellen ist dem Fachmann bekannt, und kann z.B. gemäß dem in Beispiel 2 angegebenen Verfahren durchgeführt werden. Besonders vorteilhaft ist hierbei die Subkultivierung der erfindungsgemäßen Keratinozyten bei einer maximalen Konfluenz von 80%. Die Keratinozyten lassen sich bei 35 bis 38°C, bevorzugt bei 37°C, einer Luftfeuchtigkeit von > 90%, bevorzugt 95% und einer CO₂-Sättigung von 5 bis 9% kultivieren.

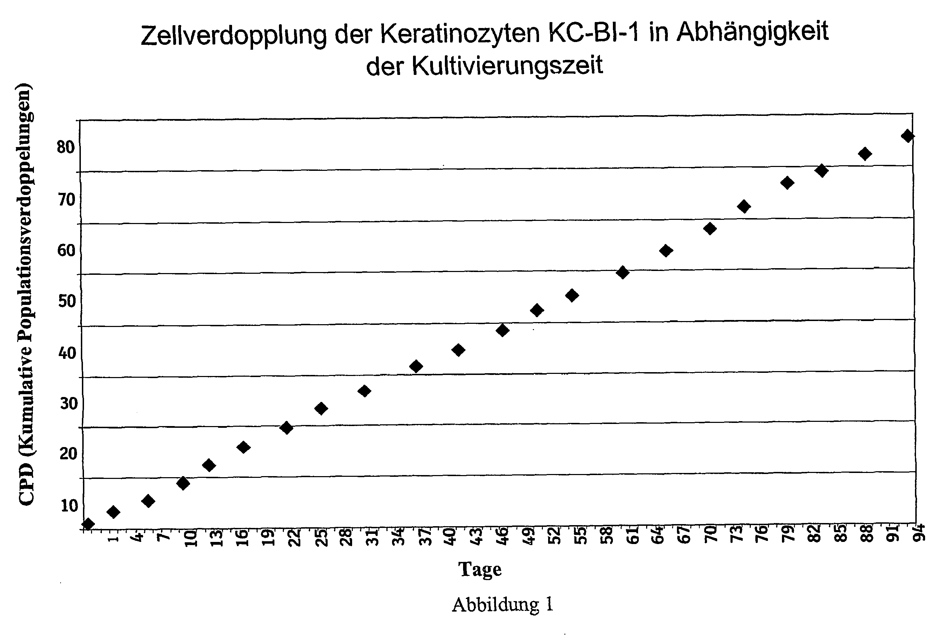

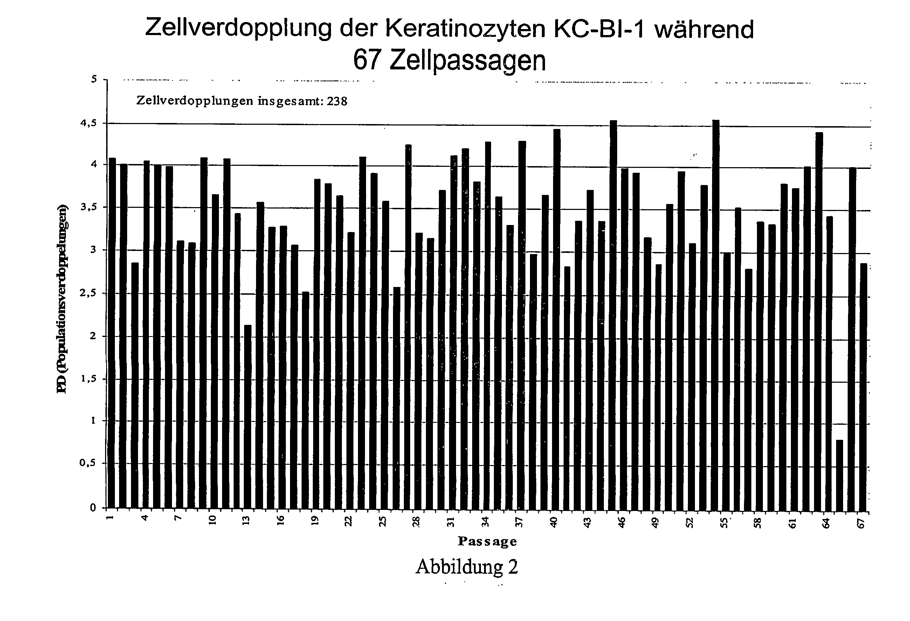

Mit dem unter Beispiel 1 dargestellten Verfahren beträgt die Verdopplungszeit der Keratinozyten zwischen 1 und 2 Tagen (Abbildung 1). Die Zellen lassen sich hierbei über zahlreiche Passagen mit annähernd konstanter Verdopplungsrate kultivieren (Abbildung 2). Die vorliegenden Erfindung ist hierbei allerdings nicht allein auf die Keratinozyten KC-BI-1 beschränkt, vielmehr ist es für einen Fachmann möglich, die Erfindung unter entsprechenden Bedingungen mit allen Keratinozyten gemäß Anspruch 1 auszuführen.

Der Ausdruck "nicht immortalisiert" meint in Bezug auf die vorliegende Erfindung, dass primär isolierte Keratinozyten sowie die hier in Kultur genommenen Keratinozyten nicht spontan transformiert sind und/oder durch dem Stand der Forschung bekannte molekularbiologische, chemische oder physikalische Verfahren transformiert worden sind. Letzteres meint, dass die Zellen weder unter Verwendung von z.B. viralen Faktoren oder Sequenzen, chemisch mutagenen Substanzen oder z.B. durch Bestrahlung oder einer Kombination aus verschiedenen Verfahren behandelt worden sind.

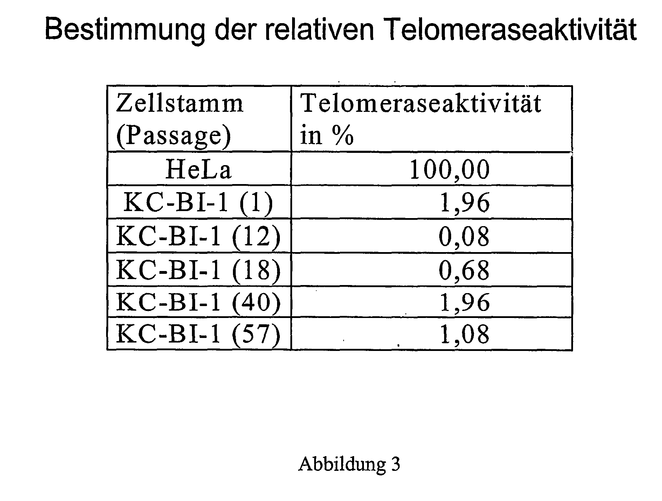

Keratinozyten, die "nicht immortalisiert sind" meint auch, dass die besagten Keratinozyten im Vergleich zu transformierten Tumorzellinien (Härle-Bachor and Boukamp, 1993) oder im Vergleich zu immortalisierten Zellienen, bevorzugt zu der Zellinie HeLa keine oder eine wesentliche geringere Telomeraseaktivität aufweist (Vgl. Abbildung 3). Dies gilt insbesondere auch im Vergleich zu der immortalisierten Keratinozytenzellinie HaCat.

Der Ausdruck "nicht immortalisiert" meint ferner, dass die besagten Keratinozyten nicht in Abwesenheit von fötalem Kälberserum und/oder nicht in Abwesenheit von Feederzellen und/oder nicht in Abwesenheit von Epidermalem Wachstumsfaktor (englisch: *epidermal groth factor*, EGF) verdoppelt werden können, wie dies beispielsweise bei immortalisierten Keratinozyten möglich ist (Schoop et al., 1999).

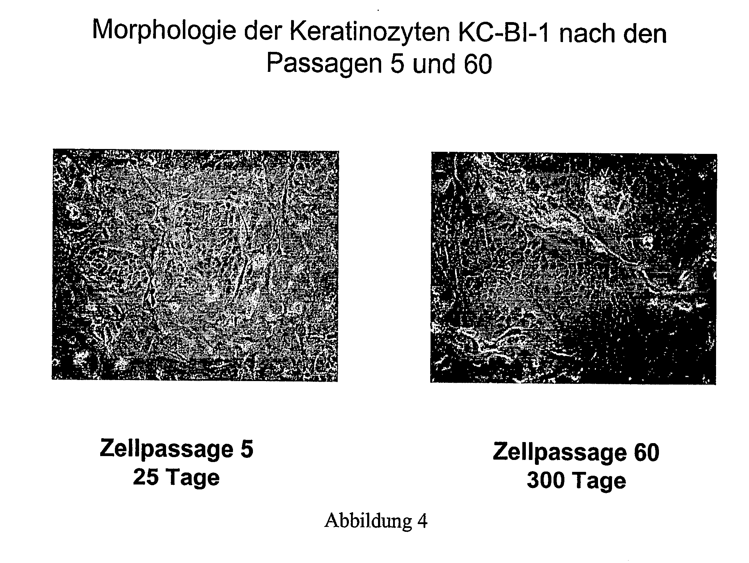

Der Ausdruck "nicht immortalisiert" meint aber auch, dass die besagten Keratinozyten ihren charakteristischen Phänotyp nicht mit zunehmender Zellverdopplung verändern (Vgl. Abbildung 4).

Ferner meint "nicht immortalisiert" auch, dass die besagten Keratinozyten, nach Transplantation auf Nacktmäuse, bevorzugt BALB/c Mäuse ein normales Differenzierungsprofil zeigen. "Normales Differenzierungspotential" meint hier die Fähigkeit der Keratinozyten, sich zu terminal differenzierten Keratinozyten zu entwickeln und suprabasale epidermale Schichten sowie ein Stratum corneum entsprechend autologer Keratinozyten auszubilden.

Gegenstand der Erfindung sind auch solche Keratinozyten, die nicht immortalisiert sind und sich mindestens 200 mal durch *in vitro* Zellkulturverfahren verdoppeln lassen. Die Erfindung betrifft ferner Keratinozyten, die nicht immortalisiert sind und sich mindestens 250 mal durch *in vitro* Zellkulturverfahren verdoppeln lassen. Die Erfindung betrifft darüber hinaus auch solche Keratinozyten, die nicht immortalisiert sind und sich mindestens 300 mal durch in *vitro* Zellkulturverfahren verdoppeln lassen.

Die vorliegende Erfindung ermöglicht die vorteilhafte Vermehrung der besagten Keratinozyten ausgehend von nur einem Spender. Ausgehend von einer Spende lassen sich beispielsweise nach 150 Zellverdopplungen 1044, nach 250 Zellverdopplungen 10⁷⁷ und nach 300 Zellverdopplungen 1090 Zellen herstellen. Dadurch ermöglicht die Erfindung erstmals die Bereitstellung großer Mengen an standardisiertem Zellmaterial für die Herstellung von biologisch aktiven Wundheilverbänden mit gleichbleibender und kontrollierbarer Qualität. Eine entsprechende Menge an standardisiertem Zellmaterial lässt sich z.B. ausgehend von einer kryokonservierten Zellbank vermehren, die wiederum aus Keratinozyten hergestellt wird, die über die erfindungsgemäßen Eigenschaften verfügen.

Mit der Verwendung von standardisiertem Zellmaterial als Ausgangsmaterial für die Herstellung von biologisch aktiven Wundheilverbänden wird auch das Infektionsrisiko für die möglichen Empfänger verringert, da die Isolierung der Keratinozyten auf einen Spender beschränkt bleibt.

Damit überwindet die vorliegende Erfindung wesentliche Nachteile, die zur Zeit in der lediglich eingeschränkten Passagierbarkeit der bisher bekannten Keratinozyten besteht.

Gegenstand der Erfindung ist darüber hinaus ein Erzeugnis, das aus einem Träger besteht, der mit den erfindungsgemäßen Keratinozyten beschichtet ist. "Beschichtet" im Sinne der Erfindung meint, dass der Träger auf seiner Oberfläche partiell oder vollständig mit den erfindungsgemäßen Keratinozyten bewachsen ist. Ein partiell bewachsener Träger ist besonders geeignet, da eine kürzere Kultivierungszeit benötigt wird bis der besagte Träger zur Wundbehandlung verwendet werden kann.

Ein im Sinne der Erfindung geeigneter Träger ist **dadurch gekennzeichnet, dass** es sich um ein biokompatibles Trägermaterial handelt, das zur Herstellung eines Arzneimittels verwendet werden kann. Verwendbar sind beispielsweise hydrophobe biokompatible Trägermaterialien, wie etwa die in WO 91/13638 beschriebenen. Es können aber auch Trägermaterialien mit vorwiegend hydrophilem Eigenschaften verwendet werden.

Eine bevorzugte Ausführung der vorliegenden Erfindung besteht in der Verwendung von Trägermaterialien die aus einem Polymer aus veresterter Hyaluronsäure bestehen. Bei einer besonders bevorzugten Ausführung handelt es sich um ein Polymer aus veresterter Hyaluronsäure, bestehend aus einer perforierten Polymerfolie mit definierter Geometrie. Die Polymerfolie hat hierbei beispielweise eine Dicke von 10 bis 500 µm und ist mit Löchern mit einer Größe zwischen 10 und 1000 µm durchlöchert, wobei die Löcher eine definierte und konstante Größe haben und eine geordnete Reihe bilden, worin sie durch einen konstanten Abstand von 50 bis 1000 µm voneinander getrennt sind. Eine entsprechende Folie ist in EP 0 462 426 beschrieben. Perforierte Trägermaterialien eignen sich besonders gut, da sie kein gerichtetes Auflegen des biologisch aktiven Wundverbandes auf die Wunde erfordern. In Beispiel 3 wird die beispielhafte Herstellung einer mit den erfindungsgemäßen Keratinozyten bewachsenen perforierten Trägermatrix aus veresterter Hyaluronsäure mit definierter Geometrie beschrieben. Bei der Trägermatrix handelt es sich um ein in Deutschland unter dem Produktnamen "Laserskin" vertriebenes Produkt der Firma Fidia Advanced Biopolymers Ltd., Abano Terme, Italien.

Die besonders bevorzugte Eignung dieses Trägermaterials zur Herstellung eines biologisch aktiven Wundverbandes in Kombination mit Keratinozyten wurde bereits am Tiermodell (Lam et al., 1999) und am Menschen gezeigt (Harris et al., 1999). Neben einer verbesserten Migration und Ausdifferenzierung der epithelialen Zellen hat die aus Hyaluronsäureester bestehende Matrix einen positiven Effekt auf die Angiogenese und die Kollagenproduktion. Die zur Zeit verwendeten Wundverbände mit Hyaluronssäureester als Matrix sind allerdings mit autologen Keratinozyten und/oder komplex aufgebauten Hautäquivalenten aus Keratinozyten und Fibroblasten beschichtet. Für sie gelten somit die oben beschriebenen Nachteile aus dem Stand der Technik. Dieser Nachteil kann insbesondere durch die Verwendung der vorteilhaften erfindungsgemäßen allogenen Keratinozyten überwunden werden.

Eine weitere bevorzugte Ausführungsform der Erfindung betrifft ein Produkt bestehend aus den erfindungsgemäßen Keratinozyten zusammen mit resorbierbaren Polymeren, bestehend aus Polyester, Polycarbonate, Polyanhydride, Polyorthoester, Polydepsipeptide, Polyetherester, Polyaminosäuren oder Polyphosphazene, insbesondere aus Poly(L-lactid), Poly(D,L-lactid), Poly(L-lactid-co-D,L-lactid), Poly(glycolid), Poly(L-lactid-co-glycolid), Poly(L-lactid-co-trimethylen-carbonat) oder Poly(dioxanon). Die besagten Polymere sind dabei sowohl perforiert als auch unperforiert.

Die vorliegende Erfindung betrifft ebenfalls ein Verfahren zur Kryokonservierung der erfindungsgemäßen Keratinozyten bei einer Temperatur von -20°C bis -196°C, bevorzugt bei einer Temperatur unter -180°C. Die besagten Keratinozyten lassen sich nach standardisierten und einem dem Fachmann geläufigen Verfahren einfrieren. Als Kryoprotektant lässt sich u.a. DMSO verwenden. Darüber hinaus ist auch die Verwendung anderer Kryoprotektanten möglich, wie etwa Glycerol, Hydroxyethylstärke oder eine Kombination aus diesen beiden sowie eine Kombination dieser mit DMSO. Entsprechende Verfahren sind beispielhaft in WO 96/24018, US 5,891,617 oder auch US 5,298,417 ausgeführt.

Gegenstand der vorliegenden Erfindung ist auch die Kryokonservierung der mit den erfindungsgemäßen Keratinozyten beschichteten Träger, **dadurch gekennzeichnet dass,** die Keratinozyten mit entsprechenden Träger bei einer Temperatur von -20°C bis -196°C, bevorzugt bei -60°C bis - 80°C kryokonserviert werden. Die Kryokonservierung hat den Vorteil, dass das in großen Mengen herstellbare Erzeugnis gelagert werden und somit vor seiner medizinischen Verwendung stichprobenartig auf seine einheitliche Qualität untersucht werden kann. Die Lagerung ermöglicht letztendlich auch dass die besagten Wundheiverbände kurzfristig für medizinische Zwecke verfügbar sind.

Als Kryoprotektant für das erfindungsgemäße Erzeugnis eignet sich beispielhaft Hydroxyethylstärke in einer Konzentration von 7-13% (w/w). Möglich ist aber auch die Verwendung von DMSO oder Glycerol sowie eine Kombination aus verschiedenen Kryoprotektanten, insbesondere aus Hydroxyetylstärke, DMSO und/oder Glycerol. Ferner ist auch die Verwendung von Trehalose als Kryoprotektant möglich.

Nach einer schnellen Temperaturerniedrigung von 37°C auf -5 bis -10°C, bevorzugt -6 bis -8°C innerhalb von 2-5 min wird das erfindungsgemäße Produkt aus Träger und Keratinozyten bei entsprechender Temperatur für 15-30 min, bevorzugt für 23-26 min äquilibriert. Anschließend wird das Erzeugnis mit einer Einfrierrate von < 1°C/min, bevorzugt von 0,2 bis 0,6°C/min besonders bevorzugt von 0,4°C/min auf eine Temperatur von z.B. - 60 bis - 80°C herabgekühlt.

Beispiel 4 beschreibt beispielhaft die Kryokonservierung einer mit KC-BI-1 beschichteten Trägermatrix aus Hyaluronsäureester. Es ist aber auch möglich, dass andere Verfahren zur Kryokonservierung angewendet werden, z.B. die in WO 95/707611, WO 96/24018, EP 0 296 475 beschriebenen Verfahren, wobei die Aufzählung nicht abschließend zu verstehen ist, sondern lediglich darauf verweist, dass Verfahren zur Kryokonservierung von Produkten bestehend aus biokompatiblen Trägern und Keratinozyten zu dem gegenwärtigen Stand der Technik gehören.

Die vorliegende Erfindung bezieht sich auch auf die medizinische Verwendung der hier beschriebenen erfindungsgemäßen Keratinozyten und/oder des beschriebenen Produkts aus besagten Keratinozyten und einem Träger, insbesondere auf die Verwendung zur Behandlung von Wunden. Eine Ausführungsform der Erfindung stellt die Verwendung der erfindungsgemäßen Keratinozyten und/oder des Produkts aus besagten Keratinozyten und einem Träger bei der Behandlung von Verbrennungen und/oder Geschwüren dar. Bei den behandelbaren Verbrennungen handelt es sich bevorzugt um Verbrennungen zweiten Grades, bei den Geschwüren bevorzugt um chronische schlecht heilenden Unterschenkelgeschwüre des Typs *Ulcus cruris,* bevorzugt *Ulcus cruris venosum* oder auch um diabetischen Ulcera, zusätzlich auch um Dekubitus.

Die medizinische Verwendung schließt eine kombinierte und/oder ergänzende Verwendung der besagten Keratinozyten und/oder des erfindungsgemäßen Produkts aus Keratinozyten und Träger mit klassischen dem Fachmann bekannten Therapien mit positivem Effekt für die Wundheilung mit ein. Gemeint ist hierbei eine kombinierte und/oder ergänzende Verwendung eines oder mehreren anderen Stoff(en) mit positiver Wirkung für die Wundheilung. Beispielhaft zu nennen ist hier die ergänzende und oder kombinierte Behandlung von *Ulcus cruris venosum* mit Hydrokolloidverbänden und/oder die zusätzliche Verwendung von antimikrobiellen Substanzen, beispielsweise die Gabe von Antibiotika.

Gegenstand der Erfindung ist ebenfalls die Verwendung der erfindungsgemäßen Keratinozyten und/oder des Produkts aus einem biokompatiblen Träger und den besagten Keratinozyten zur Herstellung eines medizinischen Erzeugnisses zur Behandlung von Wunden, insbesondere zur Behandlung von Verbrennung und/oder Geschwüren, wie z.B. zur Behandlung von Verbrennungen zweiten Grades, *Ulcus cruris* (*venosum*), diabetische Ulcera oder Dekubitus.

Gegenstand der Erfindung ist ferner auch ein Verfahren zur Behandlung besagter Wunden, wobei das Verfahren dadurch gekennzeichnet ist, dass die erfindungsgemäßen Keratinozyten und/oder das erfindungsgemäße Produkt aus Keratinozyten und Träger auf die zu behandelnden Wunden aufgelegt werden/wird. Besagte Keratinozyten und besagtes Produkt können entweder frisch oder nach Kryokonservierung verwendet werden. Ein entsprechendes Verfahren zur Wundbehandlung ist unter Beispiel 5 beschrieben.

### Beschreibung der Abbildungen:

***Abb. 1******: Zellverdopplung der Keratinozyten KC-BI-1 in Abhängigkeit der Kultivierungszeit.*** Dargestellt ist die Anzahl der Zellverdopplungen (CPD =Cumulative Population Doublings) der Keratinozyten KC-BI-1 über eine Kultivierungszeit von 94 Tagen (*days*). Innerhalb der beobachteten 94 Tagen verdoppelten sich die Zellen ungefähr 75 mal. Dies entspricht einer mittleren Verdopplungszeit von 1,25 Tagen pro Zellverdopplung, oder 12,5 pro10 Tage.
***Abb. 2******: Verdopplung der Keratinozyten KC-BI-1 über einen Zeitraum von 10 Monaten*.** Dargestellt ist die Zellverdopplung der Keratinozyten KC-BI-1 über 10 Monate, angegeben als Population doublings (PD) in Abhängigkeit der Zellpassagen (Passage) 1-67.
***Abb. 3******: Bestimmung der relativen Telomeraseaktivität*.** Dargestellt ist die relative Telomeraseaktivität für die Keratinozyten KC-BI-1 nach Passage 1, 12, 18, 40 und 57 im Vergleich zur Aktivität der Zellinie HeLa. Die Abbildung zeigt für die Keratinozyten KC-BI-1 fast keine bzw. lediglich eine geringe Telomeraseaktivität im Vergleich zu der immortalisierten Zellinie HeLa.
***Abb. 4******: Morphologie der Keratinozyten KC-BI-1 nach den Passagen 5 und 60**.* Die Keratinozyten KC-BI-1 zeigen in der lichtmikroskopischen Darstellung keine morphologischen Unterschiede zwischen Zellpassage 5 (Kultivierungszeit 25 Tage) und Zellpassage 60 (Kultivierungszeit 300 Tage).

### Ausführungsbeispiele

### Beispiel 1: Verfahren zur Kultivierung der erfindungsgemäßen Keratinozyten am Beispiel der Kultur KC-BI-1 (DSM ACC2514)

### 1.Material

Keratinozyten KC-BI-1; bestrahlte 3T3-Mäusefibroblasten (Ammenzellen, engl.:*feeder-cells*, Herstellung vgl. Beispiel 2); Zellkulturmedium K/-1 (Zusammensetzung siehe unten); EDTA (0,02%); Trypsin/EDTA (0,05%/0,01%); Zellkulturflaschen (T-flask): 25 cm², 80 cm², 175 cm²

### 2.Auftauen der Zellen

### 2.1 Auftauen der Feeder-Zellen

Zellen zügig auftauen und in 5-10 ml vorgewärmtes K/1 Medium geben. Eine entsprechende Menge an *Feeder*-Zellen wird in eine geeignete Zellkulturflasche überführt und mit K/1 Medium ergänzt:

| | | |
|---|---|---|
| 25 cm² T-flask | 0,5x10⁶ Zellen | Endvolumen an Medium: 5-6ml |
| 80 cm² T-flask | 1,5x10⁶ Zellen | Endvolumen an Medium: 20ml |
| 175cm² T-flask | 3,5x10⁶ Zellen | Endvolumen an Medium: 50ml |

Die *Feeder*-Zellen können sofort oder innerhalb von 24 Std. verwendet werden.

### 2.2 Auftauen der Keratinozyten

Zellen zügig auftauen und in 5-10 ml vorgewärmtes K/1 Medium geben. Eine entsprechende Menge an Zellen zu den mit *Feeder*-Zellen vorbereiteten Zellkulturflaschen geben und mit frischem Medium auffüllen:

| | |
|---|---|
| 25 cm² T-flask | 0,15 x 10⁶ Zellen; Endvolumen an Medium: 6 - 10 mL |
| 80 cm² T-flask | 0,4 x 10⁶ Zellen; Endvolumen an Medium: 20 mL |
| 175 cm² T-flask | 1 x 10⁶ Zellen; Endvolumen an Medium: 50 mL |

### 3.0 Kultivierung

Inkubation der Zellen erfolgt bei 35-39°C, bevorzugt bei 37°C. Die Luftfeuchtigkeit beträgt >90%, bevorzugt 95% und die CO₂-Konzentration liegt bei 5-9%. Die Keratinozyten werden bei einer maximalen Konfluenz von 80% subkultiviert.

Hierzu wird der Zellkulturüberstand verworfen. Die *Feeder*-Zellen werden nach zweimaligem Spülen mit 0,02% EDTA (2-10ml) und einer Inkubation für 5-10 min bei 37°C durch anschließendes Abklopfen (oder unter Schütteln) von der Zellkulturflasche gelöst. Nachfolgend werden die Keratinozyten nach einer Trypsin/EDTA-Behandlung (0,05%/0,01%, 1-6 ml) für 5-10 min bei 37°C vorsichtig durch Abklopfen gelöst. Falls erforderlich, werden die restlichen Zellen vorsichtig mit einem Zellschaber gelöst. Die Trypsin/EDTA-Lösung wird durch Zugabe von K/1 Medium neutralisiert und die Zellen werden durch vorsichtiges Auf- und Abpipetieren vereinzelt. Die Zellaussaat erfolgt mit der unter 2.2 angegebenen Zellzahl. Das Zellkulturmedium K/1 wird an Tag 3 anschließend alle zwei Tage ausgetauscht.

### 4.0 Herstellung des K/1 Mediums

Alle Komponenten sowie die Stammlösungen werden nacheinander entsprechend der in Tabelle 1 angegebenen Reihenfolge zusammengegeben. Der Ansatz wird auf 1 liter mit WFI (engl.: *water for injection*) aufgefüllt. Anschließend wird der pH mit NaOH or HCl auf 7,0 bis 7,2 eingestellt. Die Osmolarität sollte zwischen 320-400 mOsm/kg betragen. Abschließend wird das Medium K/1 sterilfiltriert.

### 4.1 Herstellung der Stammlösungen

### Trijodothyronine

13,6mg Trijodothyronine in 1ml 0,1M NaOH lösen und 99ml hinzugeben PBS. Die gebrauchsfertige Lösung 1:100 in PBS verdünnen. Pro Liter Medium wird 1 ml dieser Lösung benötigt.

### EGF

1mg EGF in 100ml WFI lösen. Pro Liter Medium wird 1 ml dieser Lösung benötigt.

### rh-Insulin (nur löslich bei pH <4,0)

5g rh-Insulin werden in 0,9L WFI gegeben und der pH wird 6M HCl auf 2,5 eingestellt. Nachdem sich das rh-Insulin gelöst hat, den pH mit 1M NaOH auf 8,0 einstellen. Den Ansatz mit WFI auf 1 Liter auffüllen. Pro Liter Medium wird 1 ml dieser Lösung benötigt.

### 4.2 Mediumzusammentsetzung

| **Komponenten** | **Konzentration/L** |
|---|---|
| WFI (water for injection) | 0,8L |
| DMEM with glutamine | 10,035g |
| HAM's F12 | 2,66g |
| Natriumhydrogencarbonate | 3,07g |
| Na-pyruvate | 0,11g |
| Apo-Transferrin | 5mg |
| Adenine-Hemisulfate | 147,4mg |
| Forskolin (soluble in some trops of DMSO) | 2,05mg |
| Rh-Insulin-Concentrate | 1,0mL |
| Hydrocortison 10mM | 0,11mL |
| Trij odothyronine-stocksolution | 1,0mL |
| EGF-stocksolution | 1,0mL |
| Phenol red | 8,1mg |
| FCS (2-10%) | 20-100mL |
| 6M HCl | as required |
| 40% NaOH | as required |
| WFI | add to1,0L |

Die Kultivierung der Zellen kann aber auch ausgehend von einem frischem Biopsiematerial erfolgen, beispielsweise ausgehend von dem epidermalen Anteil einer Vorhaut. Die Primärisolierung der undifferenzierten, proliferierenden Keratinozyten kann hierbei nach dem von Rheinwald und Green 1975 beschriebenen Verfahren durchgeführt werden.

Als *Feeder*-Zellen können die unter Beispiel 2 beschriebenen Zellen verwendet werden. Darüber hinaus ist auch die Verwendung anderer, letaler Fibroblasten vorstellbar, bevorzugt die Verwendung von anderen murinen Fibroblasten, besonders bevorzugt Abkömmlinge der Zellinie 3T3.

### Beispiel 2: Herstellung von bestrahlten 3T3 Feeder-Zellen zur Kultivierung von Keratinozyten

### 1. Material

Murine 3T3 Fibroblasten (z.B. ATCC CCL 92, *3T3-Swiss albino, contact-inhibited fibroblasts*) zu beziehen bei der American Type Culture Collection (ATCC), 10801 University Boulevar, Manassas, VA, USA, DMEM+10% Fötales Kälberserum (*FCS*); PBS; 0,2% Trypsin Lösung; 0,04% EDTA Lösung; Zellkulturflaschen (T-flasks): 25 cm², 80 cm², 175 cm²

### 2. Auftauen der Zellen

Zellen zügig auftauen und in 5-10 ml vorgewärmtes Medium geben. DMSO-haltiges Medium nach Zentrifugation entfernen. Zellen in 5-10 ml Medium suspendieren. Nach Bestimmung der Zellzahl werden die Zellen mit einer Dichte von 10³ to 10⁴ Zellen/cm² in geeignete Zellkulturflaschen ausgesät. Die Inkubation erfolgt bei 35 - 39°C, bevorzugt bei 37°C. Die Luftfeuchtigkeit beträgt > 90%, bevorzugt 95% und die CO₂-Konzentration liegt bei 5-9%.

### 3. Kultivierung der Zellen

Die Zellen werden täglich auf ihr Wachstum hin untersucht. Die Zelldichte sollte eine maximale Konfluenz von 70-80% nicht überschreiten. Die Subkultivierung erfolgt erfahrungsgemäß alle 2 bis 4 Tage. Hierzu wird das Medium verworfen und die Zellen mit einer geeigneten Menge einer 1:2 Mischung an EDTA und Trypsin (0,5-5 ml) gewaschen. Anschließend werden die sich ablösenden Zellen in 3,5-20 ml Medium aufgenommen. Die Zellen werden mit einer Dichte von 10³ to 10⁴ Zellen/cm² neuausgesät.

### 4. Bestrahlung der Feeder-Zellen

Die Bestrahlung der *Feeder*-Zellen erfolgt mit einer Dosis von ungefähr 60 Gy (6000 rad in einer ¹³⁷Cs-Quelle).

Sowohl die bestrahlten als auch die unbestrahlten Zellen lassen sich nach Standardprotokollen in flüssigen Stickstoff kryokonservieren und über längere Zeit aufbewahren.

### Beispiel 3: Verfahren zur Beschichtung einer Trägermatrix, hier Laserskin, mit Keratinocyten der Kultur KC-BI-1 (DSM ACC2514)

Nachfolgend wird beispielhaft die Herstellung des erfindungsgemäßen biologisch aktiven Wundheilverbandes beschrieben. Der hier beschriebene Wundheilverband besteht aus den findungsgemäßen Keratinozyten KC-BI-1 und Laserskin, einer bioresorbierbaren Trägermatrix aus Hyaluronsäureester.

Die Erfindung ist dabei nicht auf die hier beschriebene Kombination beschränkt. Vielmehr können alle Keratinozyten zur Beschichtung verwendet werden, die über die erfinderischen Eigenschaften gemäß der Ansprüche 1-8 verfügen.

Darüber hinaus ist auch die Verwendung anderer geeigneter Trägermatrices denkbar, vorausgesetzt es handelt sich um ein biokompatibles Trägermaterial, das zur Herstellung eines Arzneimittels verwendet werden kann. Verwendbar sind beispielsweise hydrophobe biokompatible Trägermaterialien, wie etwa die in WO 91/13638 beschriebenen. Daneben können aber auch Trägermaterialien mit vorwiegend hydrophilen Eigenschaften verwendet werden.

Eine weitere bevorzugte Ausführung der Erfindung liegt in der Verwendung der erfindungsgemäßen Keratinozyten zusammen mit resorbierbaren Polymeren, bestehend aus Polyester, Polycarbonate, Polyanhydride, Polyorthoester, Polydepsipeptide, Polyetherester, Polyaminosäuren oder Polyphosphazene, insbesondere aus Poly(L-lactid), Poly(D,L-lactid), Poly(L-lactid-co-D,L-lactid), Poly(glycolid), Poly(L-lactid-co-glycolid), Poly(L-lactid-co-trimethylen-carbonat) oder Poly(dioxanon) sowie die Verwendung von perforierten Folien, bestehend aus den besagten Polymeren.

### 1. Material

K/1-Medium (vgl. Beispiel 1); PBS, 0,04% EDTA (wird mit PBS auf 0,02% verdünnt); Trypsin/EDTA (0,05%/0,01%); sterile Rouxschalen (T 25 cm², T 80 cm², T 175 cm^{2,}),Laserskin (Fa. Fidia Advanced Biopolymers sr1, Abano Terme, Italien) in 144x21 Petrischalen (145cm² Fläche); 3T3-*Feeder*-Zellen; erfindungsgemäße Keratinozyten wie zum Beispiel KC-BI-1

### 2. Kultivierung des biologisch aktiven Wundheilungsverbandes

### 2.1. Material

Bestrahlte *Feeder-Zellen,* z.B. die unter Beispiel 2 ausgeführten murinen 3T3 Fibroblasten; erfindungsgemäße Keratinozyten aus der Stammhaltung; 8,5cm x 8,5cm große Laserskinstücke in einer Petrischale (Fertigprodukt); K/2-Medium

### 2.2. Aussaat der 3T3 Feeder-Zellen

Die *Feeder*-Zellen, hergestellt gemäß des Ausführungsbeispiels 2, werden mit einer Einsaatdichte von ca. 15.000 bis 25.000 Zellen/cm² (entspricht ungefähr 3x10⁶ Zellen/Petrischale) auf Laserskin aufgebracht. Die Petrischale wird anschließend bei >90% Luftfeuchtigkeit und 5-11 % CO₂, vorzugweise 7-9% in einem 37°C Brutschrank inkubiert bei 35 bis 37°C inkubiert. Die Keratinozyten werden entweder am selben Tag oder spätestens am nächsten Tag (nach 24 Std.) auf den *Feeder*-Zellrasen gesät.

### 2.3. Aussaat und Kultivierung der Keratinozyten

Mit den erfindungsgemäßen Keratinozyten werden die biologisch aktiven Wundheilverband hergestellt. Die Subkultivierung der Keratinozyten kann beispielhaft wie nachfolgend beschrieben erfolgen: Die subkonfluenten Kulturen werden einmal mit 0,02% EDTA gespült (80 cm² Rouxschale: 8 mL; 175 cm² Rouxschale: 10 ml). Anschließend werden die *Feeder*-Zellen mit 0,02% EDTA für 5-10 min bei 37°C inkubiert (80 cm² Rouxschale: 8 mL; 175 cm² Rouxschale: 10 ml). und durch vorsichtiges Schütteln abgelöst.

Die Keratinozyten werden entsprechend Ausführungsbeispiel 1 mit einem Trypsin/EDTA Gemisch (0,05%/0,01%) gelöst (80 cm² Rouxschale: 2-3 mL; 175 cm² Rouxschale: 5-6 ml), anschließend in Zellkukturmendium aufgenommen (80 cm² Rouxschale: 7-8 mL; 175 cm² Rouxschale: 14-15 ml) und durch vorsichtiges Auf- und Abpipetieren vereinzelt.

Auf die mit *Feeder*-Zellen vorbereitete Laserskin-Folie werden die erfindgungsgemäßen Keratinozyten mit einer Aussatdichte von ca. 15.000 bis 25.000 Zellen/cm² (entspricht ungefähr 3x10⁶ Zellen/Petrischale) aufgebracht. Anschließend werden die Zellen bis zu einer Konfluenz von 30 - 100%, bevorzugt von 80 - 100% bei 35 - 39°C, bevorzugt bei 37°C inkubiert. Die Luftfeuchtigkeit beträgt > 90%, bevorzugt 95% und die CO₂-Konzentration liegt bei 5 - 9%.

### Beispiel 4: Verfahren zur Kryokonservierung des erfindungsgemäßen biologisch aktiven Wundheilverbandes

Nachdem die Keratinozyten die Trägermatrix mit einer Konfluenz von 30- 100% vorzugsweise 80-100% bewachsen haben, kann das erfindungsgemäße Produkt in geeigneten Behältnissen, z.B. verschweißbaren PP-Beuteln, kontrolliert eingefroren werden. Hierzu wird Kulturmedium vorsichtig entfernt und durch 20 ml 2-6°C kaltes Einfriermedium K/2 ausgetauscht. Das Produkt wird anschließend steril verpackt und entsprechend dem nachfolgenden Programm eingefroren:

Nach einer schnellen Temperaturerniedrigung auf - 5 bis - 10°C, bevorzugt -6 bis -8°C innerhalb von 2-5 min wird das Produkt bei entsprechender Temperatur für 15-30 min, bevorzugt für 23-25 min äquilibriert. Anschließend wird das Produkt mit einer Einfrierrate von < 1°C/min, bevorzugt von 0,2 bis 0,6°C/min besonders bevorzugt von 0,4°C/min auf eine Temperatur von z.b. -60 bis -80°C herabgekühlt. Die Lagerung des Produktes erfolgt bei -60 bis -80°C.

### K/2-Einfriermedium:

### K/1-Wachstumsmedium (vgl. Beispiel 1) versetzt mit 7-13% (w/w) Hydroxyethylstärke.

### Beispiel 5: Beispielhafte Verwendung einer mit den erfindungsgemäßen Keratinozyten bewachsenen Trägermatrix zum Abdecken von Wunden am Beispiel von Ulcus cruris venosum

### 1. Transport frisch hergestellter Wundheilungsverbände

Nachdem die Keratinozyten 30-100%, bevorzugt 80-100% konfluent auf dem Laserskin bewachsen sind, wird die Kultur mit einer geeigneten Menge, vorzugsweise 30 ml, K/3-Transportmedium ein bzw. mehrmals gespült. Transportiert wird der biologisch aktive Wundheilverband in geeigneter Menge, vorzugsweise in 20 ml K/3-Transportmedium. Der Kopfraum der Petrischale wird mit einem Luftgemisch aus 5-10% CO₂ kurz begast, mit einem Klebeband z.B. Parafilm verschlossen und sofort in einer Transportbox in die Klinik gebracht.

### K/3-Transportmedium:

Wachstumsmedium K/1 (vgl. Beispiel 1) ohne Fötales Kälber Serum (*FCS*). Vorstellbar ist aber auch die Verwendung von einfachen physiologischen Salzlösungen etwa auf Phosphat-Borat-Basis, z.B. PBS, oder auch auf HEPES- (N-2-hydroxyelhylpiperazin-N'-2-ethansulfonsäure) oder MES-Basis ([2-N-morpholino]ethansufonsäure).

### 2. Transport kroykonservierter Wundheilungsverbände

Kryokonservierte Wundheilverbände können typischerweise auf Trockeneis in den Kliniken angeliefert werden. Es ist aber auch eine andere Transportform vorstellbar, sofern die Wundheilverbände bei einer Temperatur von unter - 60°C transportiert werden.

Die kryokonservierten Wundheilverbände werden zügig aufgetaut. Anschließend wird das Einfriermedium entfernt und das Verband mit K/3-Transportmedium (vgl. oben) oder einer anderen geeigneten physiologischen Lösung, z.B. Ringerlösung) ein bis mehrmals gespült.

### 3. Therapeutische Verwendung

Der Verband wird anschließend auf die Wunde aufgelegt. Bei der Verwendung nicht perforierter Trägermaterialien, ist ein gerichtetes Auflegen des Wundheilverbandes mit den Zellen in Richtung der Wunde erforderlich. Die Verwendung von perforierten Trägern, die ein beidseitiges Bewachsen der Träger mit Keratinozyten ermöglichen (z.B. Laserskin), erfordert kein gerichtetes Auflegen des erfindungsgemäßen Wundheilverbandes auf die zu behandelnde Wunde. Je nach Therapieerfolg kann die Behandlung mehrfach wiederholt werden.

### Beispiel 6: genetische Charakterisierzng der Keiatinozyfenzelle KC-BI-1 (DSMACC2514)

KC-BI-1 Zellen wurden nach der oben beschriebenen erfindungsgemäßen Methode über mehrere Passagen subkultiviert. Zellen aus Passage 4, 13 und 121 wurden anschließend einer genetischen Analyse unterzogen wobei der Längenpolymorphismus von 15 verschiedenen Genorten untersucht wurde (CSF1PO, D13S317, D16S539, D18S51, D21S11, D3S1358, D5S818, D7S820, D8S1179, FGA, Penta D, Penta E, TH01, TPOX und vWA). Die Analyse erfolgte nach einem im Stand der Technik bekannten Verfahren. Hierzu wurden die entsprechenden Allele unter Verwendung eines Test zur Bestimmung der Vaterschaft (PoewerPlex® 16 System) der Firma Promega, (Mannheim, Deutschland) entsprechend der Herstellerangaben amplifiziert. Die Allele können durch Bestimmung der Fragmentlänge identifiziert werden (Längendstandard ILS 600 ist Bestandteil des oben genannten Kits). Angaben zu Allelhäufigkeiten in der Bevölkerung sind den entsprechenden Tabellen zu entnehmen.

Die Analyse hat eine Übereinstimmung sämtlicher Allele an sämtlichen Genorten für alle analysierten Zellpassagen ergeben. Die Daten erlauben somit eine genetische Zuordnung der KC-BI-1 Zellen. Aus den Allelhäufigkeiten wurde eine Zuordnungswahrscheinlichkeit von > 99.999% ermittelt.

Bestimmung des DNA-Längenpolymorphismus:

| Marker | Spender | KC-BI-1 Passage 4 | KC-BI-1 Passage 13 | KC-BI-1 Passage 121 |
|---|---|---|---|---|
| CSF1PO | 11 | 11 | 11 | 11 |
| | 12 | 12 | 12 | 12 |
| D13S317 | 8 | 8 | 8 | 8 |
| | 13 | 13 | 13 | 13 |
| D16S539 | 11 | 11 | 11 | 11 |
| | 13 | 13 | 13 | 13 |
| D18S51 | 13 | 13 | 13 | 13 |
| | 17 | 17 | 17 | 17 |
| D21S11 | 29 | 29 | 29 | 29 |
| | 29 | 29 | 29 | 29 |
| D3S1358 | 15 | 15 | 15 | 15 |
| | 15 | 15 | 15 | 15 |
| D5S818 | 9 | 9 | 9 | 9 |
| | 11 | 11 | 11 | 11 |
| D7S820 | 9 | 9 | 9 | 9 |
| | 9 | 9 | 9 | |
| D8S1179 | 10 | 10 | 10 | 10 |
| | 14 | 14 | 14 | 14 |
| FGA | 23 | 23 | 23 | 23 |
| | 26 | 26 | 26 | 26 |
| Penta D | 11 | 11 | 11 | 11 |
| | 12 | 12 | 12 | 12 |
| Penta E | 10 | 10 | 10 | 10 |
| | 12 | 12 | 12 | 12 |
| TH01 | 8 | 8 | 8 | 8 |
| | 9,3 | 9,3 | 9,3 | 9,3 |
| TPOX | 8 | 8 | 8 | 8 |
| | 10 | 10 | 10 | 10 |
| vWA | 16 | 16 | 16 | 16 |
| | 18 | 18 | 18 | 18 |

### Literatur:

Beele H; Naeyaert JM; Goeteyn M; De Mil M; Kint A (1991): Repeated cultured epidermal allografts in the treatment of chronic leg ulcers of various origins. Dermatologica, 183 (1) 31-5.
De Luca M; Albanese E; Cancedda R; Viacava A; Faggioni A; Zambruno G; Giannetti A (1992): Treatment of leg ulcers with cryopreserved allogeneic cultured epithelium. A multicenter study. Archives of Dermatology, 128 (5) 633-8.
Härle-Bachor C; Boukamp P (1993) Telomerase activity in the regenerative basal layer of the epidermis in human skin and in immortal and carcinoma-derived skin keratinocytes. PNAS 93; 6476-6481
Falanga V et al. (1998): Rapid Healing of venous ulcers and lack of clinical rejection with an allogeneic cultured human skin equivalent. Archives of Dermatology, 134, 293-300
Harris PA; Di Franncesco F; Barisoni D; Leigh IM; Navasaria (1999): Use of hyaluronic acid and cultured autologous keratinocytes and fibroblasts in extensive burns. Lancet, 353, 35-36 Kswai K, Ikarashi Y et al. (1993): Rejection of cultured keratinocyte allografts in persensitized mice. Transplantation 56: 265-269
Lam PK; Chan ESY; Edward WH et al. (1999): Developement and evaluation of a new composite Laserskin graft. J. of Trauma: Injury, Infection and Critical Care, 47; 918-922
Lang E; Schäfer BM; Eickhoff U; Hohl HP; Kramer, M D; Maier-Reif K (1996): Rapid Normalization of epidermal integrin expression after allografting of human keratinocytes. Journal of Investigative Dermatology, 107, 423-427
Leigh IM; Navsaria H; Purkis PE; McKay I (1991): Clinical practice and biological effects of keratinocyte grafting. Annals of the Academy of Medicine, 20 (4)
Lindgren C; Marcusson JA; Toftgard R (1998): Treatment of venous leg ulcers with cryopreserved cultured allogeneic keratinocytes: a prospective open controlled study. British Journal of Dermatology, 139 (2) 271-5.
Maier K (1993): Transplantation von in vitro Epidermis - Chancen und Risiken. Quintessenz 3:289-304
Phillips TJ; Gilchrest BA (1989): Cultured allogenic keratinocyte grafts in the management of wound healing: prognostic factors. Journal of Dermatologic Surgery and Oncology, 15 (11)
Reinwald, JG and Green (1975): Serial cultivation of strains of human epidermal keratinocytes: the formation of keratinizing colonies from single cells. Cell 6, 331-344.
Schönfeld M; Moll I; Maier K; Jung EG (1993): Keratinozyten aus der Zellkultur zur Therapie von Hautdefekten. Hautarzt, 44:281-289
Schopp VM; Mirancea N; Fusenig NE.(1999): Epidermal Organizationand Differentiation of HaCaT Keratinocytes in Organotypic Coculture with Human dermal Fibroblasts. J. Invest. Dermatology 112. 343-353
Tanczos E; Horch RE; Bannasch H; Andree C; Walgenbach KJ; Voigt M; Stark GB (1999): Keratinozytentransplantation und Tissue Engineering. Neue Ansatze in der Behandlung chronischer Wunden. Zentralbl Chir 124 Suppl 1, 81-86
Teepe RGC; Roseeuw DI; Hermans J.; Koebrugge EJ; Altena T; De Coninck A; Ponec M; Jan Vermeer B (1993): Randomized trial comparing cryopreserved cultured epidermal allografts with hydrocolloid dressings in healing chronic venous ulcers. Journal of the American Academy of Dermatology, 29/6 (982-988).
Wagner G; Horch R; Debus M; Tanczos E; Jiao XJ; Saied S; Stark G.B. (1997): Human keratinocytes cultured subconfluent on esterified hyaluronic acid membranes for resurface full thickness nude mice wounds. European Journal of Cell Biology, 74, No.47, pp. 61.

## Patentansprüche

1. Keratinozyten **dadurch gekennzeichnet, dass** sie nicht immortalisiert sind und sich durch *in vitro* Zellkulturverfahren mindestens 150 mal verdoppeln lassen und dass es sich dabei um Zellen aus der Kultur KC-BI-1 (DSM ACC 2514) handelt, oder um hiervon abgeleitete Keratinozyten, wobei die besagten Keratinozyten
a. nicht in Abwesenheit von Fötalem Kälber Serum und/oder
b. nicht in Abwesenheit von *Feeder*-Zellen und/oder
c. nicht in Abwesenheit von Epidermalem Wachstumsfaktor (EGF) verdoppelt werden können,
und wobei die Telomeraseaktivität der besagten Keratinozyten im Vergleich zu der Zellinie HaCaT um mindestens Faktor 2 niedriger ist,
und wobei abgeleitete Keratinozyten Zellen und Kulturen sind, die durch Subpassagieren und / oder Subklonieren der ursprünglichen Kultur KC-BI-1 (DSM ACC 2514) generiert werden und/ oder generiert werden können.

2. Produkt bestehend aus einem Träger, der mit Keratinozyten gemäß Anspruch 1 beschichtet ist,
a. wobei der Träger partiell mit Keratinozyten bewachsen ist; oder
b. wobei der Träger vollständig mit Keratinozyten bewachsen ist.

3. Produkt gemäß Anspruch 2, **dadurch gekennzeichnet, dass** es sich bei dem Träger um ein biokompatibles Trägermaterial handelt, das zur Herstellung eines Arzneimittels verwendet werden kann.

4. Produkt gemäß Anspruch 3, **dadurch gekennzeichnet, dass** das Trägermaterial eine hydrophobe oder hydrophile biodegradierbare Membran ist.

5. Produkt gemäß der Ansprüche 3 oder 4, **dadurch gekennzeichnet, dass** es sich bei dem Träger um ein Polymer aus veresterter Hyaluronsäure handelt, bevorzugt um ein perforierte Polymerfolie mit definierter Geometrie,wobei die Polymerfolie eine Dicke von 10 bis 500 µm und mit Löchern mit einer Größe zwischen 10 und 1000 µm durchlöchert ist, wobei die Löcher eine definierte und konstante Größe haben und eine geordnete Reihe bilden, worin sie durch einen konstanten Abstand von 50 bis 1000 µm voneinander getrennt sind.

6. Produkt gemäß der Ansprüche 3 oder 4, **dadurch gekennzeichnet, dass** es sich bei dem Trägermaterial um Polyester, Polycarbonate, Polyanhydride, Polyorthoester, Polydepsipeptide, Polyetherester, Polyaminosäuren oder Polyphosphazene handelt,
a. insbesondere um Poly(L-lactid), Poly(D,L-lactid), Poly(L-lactid-co-D,L-lactid), Poly(glycolid), Poly(L-lactid-co-glycolid), Poly(L-lactid-co-trimethylen-cabonat) oder Poly(dioxanon),
b. wobei die besagten Polymere perforiert oder
c. nicht perforiert sind.

7. Verfahren zur Kryokonservierung von Keratinozyten gemäß Anspruch 1 oder eines Produkts gemäß der Ansprüche 2-6, bei dem die Keratinozyten oder das Produkt bei einer Temperatur von -20°C bis -196°C, bevorzugt bei -60 bis -80°C kryokonserviert werden/wird.

8. Keratinozyten gemäß Anspruch 1 oder Produkt aus Keratinozyten und Träger gemäß der Ansprüche 2-6 die/das nach dem unter Anspruch 7 beanspruchten Verfahren behandelt worden sind/ist.

9. Keratinozyten gemäß der Ansprüche 1 und 8 oder Produkt gemäß der Ansprüche 2-6 zur medizinischen Verwendung.

10. Verwendung von Keratinozyten gemäß Anspruch 1 und 8 oder eines Produkts gemäß der Ansprüche 2-6 zur Herstellung eines Medikamentes zur Behandlung von Wunden.

11. Verwendung gemäß Anspruch 10, wobei es sich bei den Wunden bevorzugt um Verbrennungen und/oder Geschwüren handelt.

12. Verwendung gemäß Anspruch 11, wobei es sich bei den Wunden bevorzugt um Verbrennungen zweiten Grades handelt.

13. Verwendung gemäß Anspruch 11, wobei es sich bei den Wunden bevorzugt um chronisch schlecht heilende Unterschenkelgeschwüre des Typs *Ulcus cruris, bevorzugt Ulcus cruris venosum* handelt.

14. Verwendung gemäß Anspruch 11, wobei es sich bei den Wunden bevorzugt um diabetisch bedingten Ulcus handelt.

15. Verwendung gemäß Anspruch 11, wobei es sich bei den Wunden bevorzugt um Dekubitus handelt.

16. Verwendung gemäß der Ansprüche 11-15 in Ergänzung zur oder in Kombination mit der Verwendung eines oder mehreren anderen Stoff(en) mit positiver Wirkung für die Wundheilung.

17. Verwendung gemäß des Anspruchs 16, wobei es sich bei dem anderen Stoff um Hydrokolloidverbänden handelt.

18. Verwendung gemäß des Anspruchs 16, wobei es sich bei dem anderen Stoff um antimikrobielle Substanzen handelt, wie z.B. um Antibiotika.

## Claims

1. Keratinocytes, **characterised in that** they are not immortalised and may be replicated at least 150 times by *in vitro* cell culture methods and that they are cells from the culture KC-BI-1 (DSM ACC 2514), or keratinocytes derived therefrom, wherein said keratinocytes
a. cannot be replicated in the absence of foetal calf serum and/or
b. in the absence of feeder cells and/or
c. in the absence of Epidermal Growth Factor (EGF),
and wherein the telomerase activity of said keratinocytes is lower than that of the cell line HaCaT by at least factor 2,
and wherein derived keratinocytes are cells and cultures which are and/or may be generated by subpassaging and/or subcloning the original culture KC-BI-1 (DSM ACC 2514).

2. Product consisting of a carrier which is coated with keratinocytes according to claim 1,
a. wherein the carrier is partially colonised with keratinocytes; or
b. wherein the carrier is completely colonised with keratinocytes.

3. Product according to claim 2, **characterised in that** the carrier is a biocompatible carrier material which may be used to prepare a pharmaceutical composition.

4. Product according to claim 3, **characterised in that** the carrier material is a hydrophobic or hydrophilic biodegradable membrane.

5. Product according to claim 3 or 4, **characterised in that** the carrier is a polymer of esterified hyaluronic acid, preferably a perforated polymer film of defined geometry, wherein the polymer film has a thickness of 10 to 500 µm and is perforated with holes measuring between 10 and 1000 µm, the holes having a defined, constant size and forming an ordered row, in which they are separated from one another by a constant spacing of 50 to 1000 µm.

6. Product according to claim 3 or 4, **characterised in that** the carrier material is polyester, polycarbonates, polyanhydrides, polyorthoesters, polydepsipeptides, polyetheresters, polyamino acids or polyphosphazenes,
a. particularly poly(L-lactide), poly(D,L-lactide), poly(L-lactide-co-D,L-lactide), poly(glycolide), poly(L-lactide-co-glycolide), poly(L-lactide-co-trimethylene-carbonate) or poly(dioxanone),
b. wherein the said polymers are perforated or
c. not perforated.

7. Process for cryopreserving keratinocytes according to claim 1 or a product according to claims 2-6, wherein the keratinocytes or the product is or are cryopreserved at a temperature of -20°C to -196°C, preferably at -60 to -80°C.

8. Keratinocytes according to claim 1 or product comprising keratinocytes and carrier according to claims 2-6 which have or has been treated by the process claimed in claim 7.

9. Keratinocytes according to claims 1 and 8 or product according to claims 2-6 for medical use.

10. Use of keratinocytes according to claims 1 and 8 or of a product according to claims 2-6 for preparing a medicament for treating wounds.

11. Use according to claim 10, wherein the wounds are preferably burns and/or ulcers.

12. Use according to claim 11, wherein the wounds are preferably second degree burns.

13. Use according to claim 11, wherein the wounds are preferably chronic, difficult to heal, lower leg ulcers of the type *Ulcus cruris,* preferably *Ulcus cruris venosum*.

14. Use according to claim 11, wherein the wounds are preferably ulcers caused by diabetes.

15. Use according to claim 11, wherein the wounds are preferably decubital ulcers.

16. Use according to claims 11-15 as a supplement to or in conjunction with the use of one or more other substances with a beneficial effect on wound healing.

17. Use according to claim 16, wherein the other substance is a hydrocolloid dressing.

18. Use according to claim 16, wherein the other substance is an antimicrobial substance such as e.g. an antibiotic.

## Revendications

1. Kératinocytes, **caractérisés en ce qu'**ils ne sont pas immortalisés et peuvent être multipliés au moins 150 fois par un procédé de culture cellulaire *in vitro* et **en ce qu'**il s'agit de cellules de la culture KC-BI-1 (DSM ACC 2514) ou de kératinocytes dérivés de celui-ci, lesdits kératinocytes ne pouvant pas être multipliés
a. en l'absence de sérum foetal bovin et/ou
b. en l'absence de cellules *Feeder* et/ou
c. en l'absence de facteur de croissance épidermique (EGF),
l'activité de télomérase desdits kératinocytes étant inférieure d'au moins facteur 2 par rapport à celle de la lignée cellulaire HaCaT,
et les kératinocytes dérivés étant des cellules et des cultures qui sont générées et/ou peuvent être générées par des sous-passages et/ou sous-clonages de la culture initiale KC-BI-1 (DSM ACC 2514).

2. Produit constitué d'un substrat qui est revêtu de kératinocytes selon la revendication 1,
a. le substrat étant partiellement recouvert de kératinocytes ; ou
b. le substrat étant complètement recouvert de kératinocytes.

3. Produit selon la revendication 2, **caractérisé en ce que** le substrat est un matériau de support biocompatible qui peut être utilisé pour la production d'un médicament.

4. Produit selon la revendication 3, **caractérisé en ce que** le matériau de support est une membrane biodégradable hydrophobe ou hydrophile.

5. Produit selon les revendications 3 ou 4, **caractérisé en ce que** le substrat est un polymère d'acide hyaluronique estérifié, de préférence, une feuille polymère perforée présentant une géométrie définie, la feuille polymère présentant une épaisseur de 10 à 500 µm et étant perforée de trous présentant une taille entre 10 et 1000 µm, les trous ayant une taille définie et constante et formant une rangée ordonnée, dans laquelle ils sont séparés les uns des autres par un intervalle constant de 50 à 1000 µm.

6. Produit selon les revendications 3 ou 4, **caractérisé en ce que** le matériau de support est un polyester, un polycarbonate, un polyanhydride, un polyorthoester, un polydepsipeptide, un polyétherester, des acides polyaminés ou un polyphosphazène,
a. notamment, un poly(L-lactide), un poly(D, L-lactide), un poly(L-lactide-co-D,L-lactide), un poly(glycolide), un poly(L-lactide-co-glycolide), un poly(L-lactide-co-triméthylène-carbonate) ou un poly(dioxanone),
b. lesdits polymères étant perforés ou
c. non perforés.

7. Procédé de cryoconservation de kératinocytes selon la revendication 1 ou d'un produit selon les revendications 2 à 6, dans lequel les kératinocytes ou le produit est/sont cryoconservé(s) à une température de -20° C à -196° C, de préférence, de -60 à -80° C.

8. Kératinocytes selon la revendication 1 ou produit à base de kératinocytes et substrat selon les revendications 2 à 6, qui sont/est traité(s) selon le procédé de la revendication 7.

9. Kératinocytes selon les revendications 1 et 8 ou produit selon les revendications 2 à 6 pour l'utilisation médicale.

10. Utilisation de kératinocytes selon les revendications 1 et 8 ou d'un produit selon les revendications 2 à 6 pour la production d'un médicament pour le traitement de plaies.

11. Utilisation selon la revendication 10, les plaies étant de préférence des brûlures et/ou des ulcères.

12. Utilisation selon la revendication 11, les plaies étant de préférence des brûlures du deuxième degré.

13. Utilisation selon la revendication 11, les plaies étant de préférence des ulcères de la jambe chroniques cicatrisant difficilement, du type *Ulcus cruris,* de préférence, l'ulcère variqueux.

14. Utilisation selon la revendication 11, les plaies étant de préférence un ulcère d'origine diabétique.

15. Utilisation selon la revendication 11, les plaies étant de préférence des escarres.

16. Utilisation selon les revendications 11 à 15 en complément ou en combinaison avec l'utilisation d'une ou plusieurs autre(s) substance(s) ayant un effet positif sur la cicatrisation des plaies.

17. Utilisation selon la revendication 16, l'autre substance étant des pansements hydrocolloïdaux.

18. Utilisation selon la revendication 16, l'autre substance étant des substances antimicrobiennes, telles que par exemple, des antibiotiques.
